(19) Europäisches Patentamt
European Patent Office
Office européen des brevets

(11) Publication number: **0 356 673 B1**

(12) # EUROPEAN PATENT SPECIFICATION

(45) Date of publication of patent specification: **19.05.93**

(51) Int. Cl.5: **C07D 471/04**, A61K 31/435, //(C07D471/04,235:00,221:00)

(21) Application number: **89112906.6**

(22) Date of filing: **14.07.89**

(54) **Substituted 5,6,7,8-tetrahydroimidazo[1,5-a]pyridines and process for their preparation.**

(30) Priority: **02.09.88 GB 8820730**

(43) Date of publication of application: **07.03.90 Bulletin 90/10**

(45) Publication of the grant of the patent: **19.05.93 Bulletin 93/20**

(84) Designated Contracting States: **DE GB IT**

(56) References cited: **EP-A- 0 165 904**

(73) Proprietor: **FARMITALIA CARLO ERBA S.r.L.**
**Via Carlo Imbonati 24**
**I-20159 Milano(IT)**

(72) Inventor: **Buzzetti, Franco**
**Via Gallarana 4**
**I-20052 Monza (MI)(IT)**
Inventor: **Di Salle, Enrico**
**Viale Andrea Doria 5**
**I-20124 Milano(IT)**
Inventor: **Lombardi, Paolo**
**XVIa Strada 22**
**I-20020 Cesate (MI)(IT)**

(74) Representative: **Ferrario, Vittorino**
**c/o Farmitalia Carlo Erba S.r.l., Via Carlo Imbonati 24**
**I-20159 Milano (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 356 673 B1

Rank Xerox (UK) Business Services
(3.10/3.6/3.3.1)

## Description

- The present invention relates to 5,6,7,8-tetrahydroimidazo-[1.5-a] pyridines, to a process for their preparation, to pharmaceutical compositions containing them, and to the use of said compounds for the treatment of hormone-dependent diseases, in particular cancers, in mammals.
Basic and clinical data indicate that aromatized metabolites of androgens, i.e. the estrogens, are the hormones involved in the pathogenic cellular changes associated with the growth of some hormone-dependent cancers, such as, e.g., breast, endometrial ovarian and pancreatic carcinomas. Estrogens are also involved in the pathogenesis of benign prostatic hyperplasia.
- Endogenous estrogens are ultimately formed from either androstenedione or testosterone as immediate precursors. The reaction of central importance is the aromatization of the steroidic ring A, which is performed by the enzyme aromatase. As aromatization is a unique reaction and the last in the series of steps in the biosynthesis of estrogens, it has been envisaged that an effective inhibition of aromatase, resulting from compounds able to interact with the aromatizing steps, may have useful application for controlling the amount of circulating estrogens, estrogen-dependent processes in reproduction, and estrogen-dependent tumours.

EP-A-0 165 904 discloses substituted 5,6,7,8-tetrahydroimidazo[1,5-a]pyridines wherein the 5-position carries a phenyl group. The compounds according to this document exhibit aromatase inhibiting activities.

- Non-steroidal known substances which have also been reported to be endowed with more or less selective aromatase-inhibiting action are, for example, aminoglutethimide |Ann.Surg. 187, 475 (1978); Lancet 2, 646 (1978); 4-cyclohexylaniline |Endocrinology 114, 2128 (1984)|, and 4-pyridyl-3-ethyl-2,6-piperidinedione |J.Med.Chem. 28, 200 (1985)|.

The present invention provides compounds having the following general formula (I).

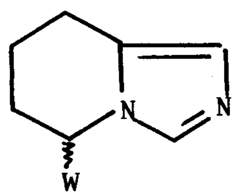

(I)

wherein W is
a 2-naphthyl or 1- or 2- or 9-anthryl radical wherein each benzene ring is unsubstituted or substituted by a substituent chosen from halogen, $C_1-C_4$ alkyl, phenyl, phenyl-$C_1-C_4$ alkyl, hydroxy, $C_1-C_4$ alkoxy, phenoxy, phenyl-$C_1-C_4$ alkoxy, formyloxy, $C_2-C_5$ alkanoyloxy, benzoyloxy, mercapto, $C_1-C_4$ alkylthio, phenylthio, phenyl-$C_1-C_4$ alkylthio, $C_1-C_4$ alkylsulfonyl, phenylsulfonyl, $C_2-C_5$ alkanoylthio, $C_2-C_5$ alkanoylamino, benzoylamino, $C_2-C_5$ alkanoyl, benzoyl, carboxy, $C_2-C_5$ alkoxycarbonyl, cyano, nitro, mono- or di-($C_1-C_4$ alkyl)carbamyl, amino, mono-($C_1-C_4$ alkyl)amino, di-($C_1-C_4$ alkyl)amino, morpholino, piperazino and $C_1-C_4$ alkyl-N-substituted piperazino, or by a quaternary ammonium radical chosen from tri-($C_1-C_4$ alkyl)amino, N-$C_1-C_4$ alkyl-substitinted morpholino, N'-$C_1-C_4$ alkyl-substituted piperazino, N''-di($C_1-C_4$ alkyl)-substituted piperazino and N',N''-di ($C_1-C_4$ alkyl)-substituted piperazino, and the pharmaceutically acceptable salts thereof.

- A halogen is iodine or bromine or fluorine, in particular chlorine.
- An alkyl group, including the alkyl moiety of the other above mentioned substituents, may be a branched or a straight chain group. A $C_1-C_4$ alkyl group is preferably methyl or ethyl, more preferably methyl.
- A $C_1-C_4$ alkoxy is preferably methoxy or ethoxy.
- A $C_2-C_5$ alkanoyloxy group is preferably acetoxy, propionyloxy or butyryloxy, more preferably acetoxy.
- A $C_1-C_4$ alkylthio group is preferably methylthio or ethylthio.
- A $C_1-C_4$ alkylsulfonyl group is preferably methylsulfonyl. A quaternary ammonium radical as defined above is, e.g.,

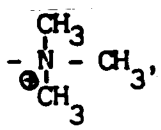, 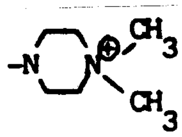, 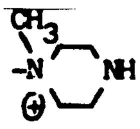, 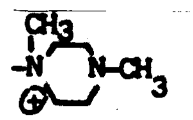 or 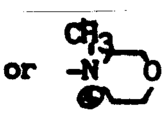.

- A $C_2-C_5$ alkanoylthio group is preferably acetylthio.
- A $C_2-C_5$ alkanoylamino group is preferably acetylamino.
- A $C_2-C_5$ alkanoyl group is preferably acetyl, propionyl or butyryl, more preferably acetyl.
- A $C_2-C_5$ – alkoxycarbonyl group is preferably methoxycarbonyl or ethoxycarbonyl.
- A mono – or di – ($C_1-C_4$ alkyl) carbamyl is preferably methylcarbamyl or dimethylcarbamyl or diethylcarbamyl, respectively.
- A mono – or di – ($C_1-C_4$ alkyl)amino group is preferably methylamino or dimethylamino or ethylamino or diethylamino respectively. A $C_1-C_4$ alkyl – N – substituted piperazino group is preferably N – methylpiperazino.
- In the formulae of this specification, a heavy solid line ( ◄ ) indicates that a substituent is in the $\beta$ – configuration, i.e. above the plane of the ring, a wavy line ( ~~ ) indicates that a substituent may be either in the $\alpha$ – configuration, i.e. below the plane of the ring, or in the $\beta$ – configuration or in both, i.e. a mixture thereof such as a racemic mixture.
- All the possible isomers of formula (I) are included within the scope of the invention, both separately and in mixture. Thus, for example, for each compound of formula (I) two distinct optical isomers, i.e. enantiomers, may exist according to configuration of the chiral carbon atom carrying the W substituent. The formula (I) is meant to cover both the enantiomers, either separately or in mixture.

The invention includes also the pharmaceutically acceptable salts of the compounds of formula (I) either with a pharmaceutically acceptable acid or base or in the form of an internal salt. The said salts are preferably the salts with pharmaceutically acceptable acids, both inorganic acids, such as e.g. hydrochloric and sulfuric, and organic acids such as e.g. citric, tartaric, maleic, succinic, methanesulfonic, and ethane sulfonic. Preferred salts are the hydrochlorides.

Preferred compounds of the invention are the compounds of formula (I) wherein W is

a 2 – naphthyl or 1 – anthrylradical in which each benzene ring is unsubstituted or substituted by a substituent chosen from halogen, hydroxy, carboxy, cyano and nitro;

and the pharmaceutically acceptable salts thereof.

Examples of specific compounds, preferred under this invention, are the following, both as single enantiomers and as mixtures of entantiomers, in particular racemic mixtures:

5 – (2′ – naphthyl) – 5,6,7,8 – tetrahydroimidazo [1.5 – a] pyridine;
5 – (4′ – chloro – 2′ – naphthyl) – 5,6,7,8 – tetrahydroimidazo [1.5 – a] pyridine;
5 – (4′ – hydroxy – 2′ – naphthyl) – 5,6,7,8 – tetrahydroimidazo [1.5 – a] pyridine;
5 – (4′ – carboxy – 2′ – naphthyl) – 5,6,7,8 – tetrahydroimidazo [1.5 – a] pyridine;
5 – (4′ – cyano – 2′ – naphthyl) – 5,6,7,8 – tetrahydroimidazo [1.5 – a] pyridine;
5 – (4′ – nitro – 2′ – naphthyl) – 5,6,7,8 – tetrahydroimidazo [1.5 – a] pyridine;
5 – (1′ – anthryl) – 5,6,7,8 – tetrahydroimidazo [1.5 – a] pyridine;
5 – (4′ – chloro – 1′ – anthryl) – 5,6,7,8 – tetrahydroimidazo [1.5 – a] pyridine;
5 – (4′ – hydroxy – 1′ – anthryl) – 5,6,7,8 – tetrahydroimidazo [1.5 – a] pyridine;
5 – (4′ – carboxy – 1′ – anthryl) – 5,6,7,8 – tetrahydroimidazo [1.5 – a] pyridine;
5 – (4′ – cyano – 1′ – anthryl – 5,6,7,8 – tetrahydroimidazo [1.5 – a] pyridine;
5 – (4′ – nitro – 1′ – anthryl) – 5,6,7,8 – tetrahydroimidazo [1.5 – a] pyridine;
and the pharmaceutically acceptable salts thereof.

According to a further object, the present invention relates to the use of a compound of formula (I), as herein defined, or a pharmaceutically acceptable salt thereof, in the preparation of a pharmaceutical composition for use as an aromatase inhibitor.

The compounds of the invention and the salts thereof can be prepared by a process comprising:

A) decarboxylating a compound of formula (II):

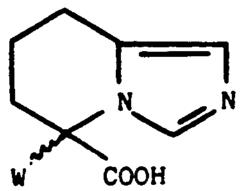

( II)

wherein W is as defined above; or

B) cyclizing a compound of formula (III):

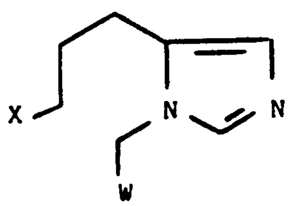

(III)

wherein W is as defined above and X is a leaving group; and, if desired, converting a compound of formula (I) into another compound of formula (I), and/or, if desired, salifying a compound of formula (I) and/or, if desired, obtaining a free compound from a salt and/or, if desired, separating a mixture of isomers of compounds of formula (I) into the single isomers.

The decarboxylation of a compound of formula (II) in order to obtain a compound of formula (I) may be carried out according to known methods.

Preferably high temperatures are applied ranging from about 100° to about 250°C. The reaction is performed at normal or high pressure e.g. up to about 50 Atm., depending on the solvent used. Suitable inert organic solvents are for instance alcohols like ethylene glycol, dipolar aprotic solvents like dimethyl-formamide or methyl sulfoxide or ethers like diglyme. Preferably the decarboxylation is carried out in the presence of an inorganic acid like hydrochloric acid or respectively the salified form is used, e.g. the hydrochloride salt.

Alternatively the decarboxylation is carried out by heating a compound of formula (II) at temperatures ranging from about 100 to about 230°C in a high boiling heterocyclic base like quinoline. The reaction can also be carried out in the presence of metallic copper at temperatures ranging from about 50 to about 200°C.

- The leaving group X in a compound of formula (III) is preferably an esterified hydroxy group, e.g. acetoxy, mesyloxy, benzenesulfonyloxy or p-toluenesulfonyloxy, or in particular a halogen atom like chlorine or bromine.

The cyclization is performed in the presence of a base. A suitable base is e.g. an alkali metal hydroxyde, like sodium or potassium hydroxide, a bicyclic amidine like 1,5-diazabicyclo [5.4.0] undec-5-ene, or preferably a $C_1-C_6$ alkoxyde like potassium methoxide, potassium ethoxide or potassium tert-butoxide, an alkali metal amide like lithium diisopropylamide or an alkali metal hydride like sodium hydride.

The cyclization is carried out in an aprotic organic solvent, e.g. an ether like ethyl ether, dioxane or tetrahydrofuran; or an amide like dimethylformamide, or in mixtures of these solvents.

The reaction temperature ranges from about -60°C to about +60°C, preferably from about -10°C to room temperature.

- Optional conversions of a compound of formula (I) into another compound of formula (I) include for example, the conversion of a compound of formula (I) with carboxyl substituent into a compound with alkoxycarbonyl substituent. This conversion may be carried out by known methods e.g., by treatment of the acid with an excess a suitable $C_1-C_4$ alkanol, such as methanol or ethanol in the presence of an anhydrous mineral acid like hydrogen chloride or sulfuric acid at temperature ranging from room temperature to reflux temperature.
- The conversion of hydroxy substituent into a $C_1-C_4$ alkoxysubstituent in the compounds of formula (I) may be carried out, e.g, by treating the phenolic compound first with a suitable base like sodium hydride or sodium amide and then with an alkylating agent like dimethyl sulfate in a suitable solvent like benzene at temperature ranging from about 20° to about 100°C.

- The introduction of a cyano substituent into an unsubstituted compound of formula (I) may be carried out according to known methods, e.g., by reaction with cyanogen chloride or bromide or with trichloroacetonitrile.
- The conversion of a halogen substituent into a cyano substituent in the compounds of formula (I) may be carried out, e.g., by treating the halide with an inorganic cyanide like sodium cyanide or copper (I) cyanide in a suitable solvent like pyridine, quinoline or dimethylformide at high temperature, preferably reflux temperature.
- The conversion of a nitro substituent into an amino substituent in the compounds of formula (I) may be carried out according to known methods, e.g., by hydrogenation of the nitro compound in the presence of hydrogenation catalyst like palladium on activated carbon in a suitable solvent like methanol or ethyl acetate.
- Conventional methods may be used for salifying a compound of formula (I) and for obtaining a free compound of formula (I) from a salt thereof, and standard procedures, such as, e.g., fractional crystallization and chromatography may be followed as well for separating a mixture of isomers of compounds of formula (I) into the single isomers.

Preparation of the intermediates

- A compound of formula (II) may be prepared by cyclization of a compound of formula (IV)

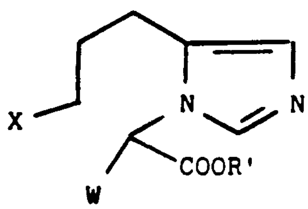

(IV).

wherein W and X are as defined above and R′ is alkyl or aralkyl, preferably lower alkyl or phenyl-lower alkyl, followed by cleavage of the cyclized ester in order to obtain the free acid (R′ = H).

The cyclization may be carried out as described above under procedure B). The resulting cyclized ester is cleaved by known methods for instance by the method described by P.G. Gassman et al., in J.Org.Chem. 42, 918 (1977).

- Some of the compounds of formula (IV) can be prepared by known methods, e.g. by transforming suitably a compound of formula (V)

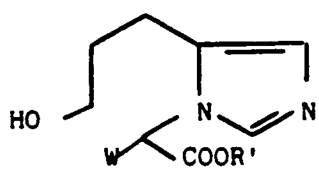

(V)

wherein W and R′ are as defined above.

For example a compound of formula (IV) wherein X is halogen, e.g. chlorine, can be obtained by reacting compound (V) e.g. with thionyl chloride in an inert solvent like dichloromethane at temperatures ranging from about 0° to about 80° C. However, other known halogenation methods for alcohols can be applied.

A compound of formula (IV) wherein X is $OSO_2Me$ can be prepared e.g. by reacting compound (V) with mesyl chloride in the presence of a base like pyridine in a suitable inert solvent at temperatures ranging from 0° to 100° C.

A compound of formula (IV) wherein X is respectively $OSO_2C_6H_5$ or $OSO_2C_6H_4Me$ can be prepared in an analogous manner.

A compound of formula (IV) wherein X is OCOMe may be obtained e.g. by reacting compound (V) with acetyl chloride or acetic anhydride in the presence of a base like pyridine or triethylamine in a suitable solvent at temperatures ranging from 0° to 100° C.

– Some of the compounds of formula (V) are known, the unknown ones can be prepared by known methods, e.g. by reaction of a compound of formula (VI)

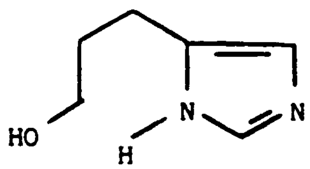

(VI)

in which the NH group is blocked by a conventional amino blocking group like dimethylcarbamyl and the hydroxy group is protected by a conventional hydroxy protecting group like trimethylsilyl with a compound of formula (VII)

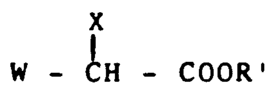

(VII)

wherein W, X and R′ are as defined above.

– Compound (VI) is a known compound. Conveniently it can be prepared according to G.A.A. Kivits et al., in J.Het.Chem. 12, 577 (1975).

– Compounds (VII) are known compounds or can be prepared according to well known methods.

Some of the compounds of formula (III) are known, the unknown ones can be prepared by known methods, e.g. by transforming a compound of formula (VIII)

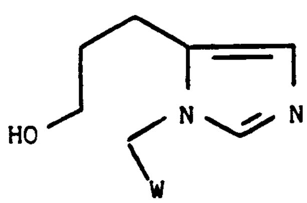

(VIII)

wherein W is as defined above. For this purpose the same methods used for the preparation of compounds of formula (IV) can be applied.

Some of the compounds of formula (VIII)are known, the unknown ones can be prepared by known methods, e.g. by reaction of a compound of formula (VI) suitably protected with a compound of formula (IX)

$W - CH_2 - X$ (IX)

wherein W and X are as defined above. The same methods as used for the preparation of compounds of formula (V) can be applied.

– Compounds of formula (IX) are known or can be prepared by well known methods.

When in the compounds of the invention, and in the intermediates thereof, groups are present which need to be protected before submitting them to the hereabove illustrated reactions, these groups may be protected by conventional methods before the reaction takes place and then deprotected according to known procedures at the end of the reaction.

The compounds of the present invention are inhibitors of the biotransformation of androgens into estrogens, i.e. they are aromatase inhibitors.

The inhibition of aromatase was demonstrated by employing the in vitro assay which utilizes the aromatase enzyme system isolated from the microsomal fraction of human placental tissue, according to standard procedure. The assay of Thompson and Siiteri [F.A. Thompson and P.K. Siiteri, J. Biol. Chem. 249, 5364, 1974] which determines the rate of aromatization as measured by the liberation of $^3H_2O$ from 4–

$[1\beta,2\beta-{}^{3}H]$androstene-3,17-dione was used.

All incubations were carried out in a shaking water bath at 37°C in air in 10 mM potassium phosphate buffer, pH 7.5, which contained 100 mM Kcl, 1 mM EDTA and 1 mM dithiothreitol.

The experiments were carried out in 1 ml incubation volume containing 50 nM 4-$[{}^{3}H]$androstenedione, various concentrations of the inhibitors, 100 $\mu$M NADPH and 0.05 mg of microsomal proteins. After 15 minutes of incubation the reaction was stopped by the addition of chloroform (5 ml).

After centrifugation at 1500x g for 5 minutes, aliquots (0.5 ml) were removed from the water phase for determination of ${}^{3}H_2O$ formed.

The concentration of each compound required to reduce control aromatase by 50% ($IC_{50}$) was determined by plotting % inhibition versus log of inhibitor concentration. The new compounds, incubated at various concentrations, showed a relevant aromatase inhibitory activity. By virtue of their ability to inhibit aromatase and, consequently, to reduce estrogen levels, the new compounds are useful in the treatment and prevention of various estrogen dependent diseases in mammals, i.e. breast, endometrial, ovarian and pancreatic cancers, gynecomastia, benign breast disease, endometriosis, polycystic ovarian disease and precocious puberty. Another application of the compounds of the invention is in the therapeutic and/or prophylactic treatment of prostatic hyperplasia, a disease of the estrogen dependent stromal tissue. The new compounds can find also use for the treatment of male infertility associated with oligospermia and for female fertility control, by virtue of their ability to inhibit ovulation and egg nidation. In view of their high therapeutic index, the compounds of the invention can be used safely in medicine. For example, the approximate acute toxicity ($LD_{50}$) of the compounds of the invention in the mouse, determined by single administration of increasing doses and measured on the seventh day after the treatment was found to be negligible.

The compounds of the invention can be administered in a variety of dosage forms, e.g. orally, in the form of tablets, capsules, sugar or film coated tablets, liquid solutions or suspensions; rectally, in the form of suppositories; parenterally, e.g. intramuscularly, or by intravenous injection or infusion.

The dosage depends on the age, weight, conditions of the patient and administration route; for example the dosage adopted for oral administration to adult humans may range from about 10 to about 150-200 mg pro dose, from 1 to 5 times daily.

The invention includes pharmaceutical compositions comprising a compound of the invention in association with a pharmaceutically acceptable excipient (which can be a carrier or diluent).

The pharmaceutical compositions containing the compounds of the invention are usually prepared following conventional methods and are administered in a pharmaceutically suitable form.

For example the solid oral forms may contain, together with the active compound, diluents, e.g., lactose, dextrose, saccharose, cellulose, corn starch or potato starch; lubricants, e.g. silica, talc, stearic acid, magnesium or calcium stearate, and/or polyethylene glycols; binding agents, e.g. starches, arabic gums, gelatin, methylcellulose, carboxymethylcellulose or polyvinyl pyrrolidone; disaggregating agents, e.g. a starch, alginic acid, alginates or sodium starch glycolate; effervescing mixtures; dyestuffs, sweeteners; wetting agents, such as lecithin, polysorbates, laurylsulphates; and, in general, non-toxic and pharmacologically inactive substances used in pharmaceutical formulations. Said pharmaceutical preparations may be manufactured in known manner, for example, by means of mixing, granulating, tabletting, sugar-coating, or film-coating processes.

The liquid dispersions for oral administration may be e.g. syrups, emulsions and suspensions.

The syrups may contain as carrier, for example, saccharose or saccharose with glycerine and/or mannitol and/or sorbitol.

The suspensions and the emulsions may contain as carrier, for example, a natural gum, agar, sodium alginate, pectin, methylcellulose, carboxymethylcellulose, or polyvinyl alcohol.

The suspensions or solutions for intramuscular injections may contain, together with the active compound, a pharmaceutically acceptable carrier, e.g. sterile water, olive oil, ethyl oleate, glycols, e.g. propylene glycol, and if desired, a suitable amount of lidocaine hydrochloride.

The solutions for intravenous injections or infusions may contain as carrier, for example, sterile water or preferably they may be in the form of sterile, aqueous, isotonic saline solutions.

The suppositories may contain together with the active compound a pharmaceutically acceptable carrier, e.g. cocoa-butter, polyethylene glycol, a polyoxyethylene sorbitan fatty acid ester surfactant or lecithin.

The following examples illustrate but do not limit the invention.

Example 1

5-(2′-naphthyl)-5,6,7,8-tetrahydroimidazo [1.5-a] pyridine [I, W = 2′-naphthyl]

A solution of 5-(2′-naphthyl)-5-carboxy-5,6,7,8-tetrahydroimidazo [1.5 a] pyridine hydrochloride (329 mg,1 mmol) in ethylene glycol (3 ml) is heated for 4 hours at 200°C under nitrogen. To the cooled mixture potassium carbonate solution is added, the raw product extracted with chloroform, the organic layer dried and evaporated under vacuum.

The resulting residue is chromatographed on silica gel using choloroform/methanol 1% as eluant to give pure title compound in 70% yield (174 mg).
Elemental analysis found % (calculated %):
C 82.15 (82.23), H6.35(6.50), N 11.15 (11.28)
NMR($CDCl_3$ δ) : 1.80-2.40 (4H,m), 3.05(2H,t), 5.38(1H,t), 6.90(1H,s), 7.52(1H,s), 7.10-8.00 (7H,m).

In analogous fashion the following compounds can be prepared:
5-(4′-chloro-2′-naphthyl)-5,6,7,8-tetrahydroimidazo [1.5-a] pyridine;
5-(4′-hydroxy-2′-naphthyl)-5,6,7,8-tetrahydroimidazo [1.5-a] pyridine;
5-(4′-cyano-2′-naphthyl)-5,6,7,8-tetrahydroimidazo-[1.5-a]pyridine;
5-(4′-carboxy-2′-naphthyl)-5,6,7,8-tetrahydroimidazo-[1.5-a] pyridine;
5-(1'-anthryl)-5,6,7,8-tetrahydroimidazo [1.5-a]-pyridine;
5-(4'-hydroxy-1'anthryl)-5,6,7,8-tetrahydroimidazo [1.5-a] pyridine;
5-(4'-cyano-1'-anthryl)-5,6,7,8-tetrahydroimidazo [1.5-a] pyridine; and
5-(4'-carboxy-1'-anthryl)-5,6,7,8-tetrahydroimidazo [1.5-a] pyridine.

Example 2

5-(1'-anthryl)-5,6,7,8-tetrahydroimidazo [1.5-a] pyridine [I, W = 1'anthryl].

To an ice cooled solution of 1-(1'anthranylmethyl)-5-(3'-chloro propyl)-IH-imidazole (335 mg, 1 mmol) in dry tetrahydrofuran (3 ml) is added portionwise potassium tert-butoxide (336 mg, 3 mmol) and the resulting reaction mixture stirred for further 6 hours at room temperature under nitrogen. Then the reaction mixture is concentrated under vacuum, the pH regulated to 8-10 by addition of aqueous acetic acid under cooling and the raw product extracted with chloroform. The organic layer is separated, dried and evaporated under vacuum. Finally the resulting residue is chromatographed on silica gel using chloroform/methanol 1% as eluant. Thus 150 mg (50% yield) of pure title compound is obtained.
Elemental analysis found % (calculated %):
C 84.35 (84.53), H 6.15 (6.08), N 9.31 (9.39).
NMR ($CDCl_3$ δ): 1.85-2.35 (4H,m), 3.00 (2H,t), 5.30 (1H,t), 6.85 (1H,s), 7.45(1H,s),7.20-8.20 (9H,m).

In analogous fashion the following compounds can be prepared:
5-(2'-naphthyl)-5,6,7,8-tetrahydroimidazo [1.5-a] pyridine;
5-(4'-nitro-2'-naphthyl)-5,6,7,8-tetrahydroimidazo [1.5-a] pyridine;
5-(4'-chloro-1'-anthryl)-5,6,7,8-tetrahydroimidazo [1.5-a] pyridine; and
5-(4'-nitro-1'-anthryl)-5,6,7,8-tetrahydroimidazo [1.5-a] pyridine.

Example 3

5-(2'-naphthyl-5-carboxy-5,6,7,8-tetrahydroimidazo [1.5-a] pyridine (II, W = 2'-naphthyl)

To an ice cooled solution of 1-(2'-naphthylethoxycarboxylmethyl)-5-(3'-chloropropyl)-1H-imidazole (357 mg, 1 mmol) in dry tetrahydrofuran (5 ml) is added portionwise potassium tert-butoxide (224 mg, 2 mmol) and the resulting reaction mixture stirred for further 2 hours at room temperature under nitrogen. Thereupon 2 mmol of glacial acetic acid is added under cooling and the mixture concentrated under vacuum. The residue is taken up with ethyl acetate and potassium carbonate solution and two phases separated. The alkaline aqueous phase contains a small amount of cyclized free acid which can be isolated by filtering off the precipitate formed after neutralization with acid.

The organic phase is washed with water, dried over sodium sulfate and evaporated under vacuum. Thus 288 mg of raw 5-(2′-naphthyl)-5-ethoxycarbonyl-5,6,7,8-tetrahydroimidazo [1.5-a] pyridine is obtained (90% yield).
NMR ($CDCl_3$ δ ): 1.30(3H,t),1.30-3.00(6H,m), 4.32(2H,q)

6.88(1H,s),7.52(1H,s),7.00 – 7.50 (7H,m)

- The above obtained ester (288 mg) is dissolved in tetrahydrofuran (5 ml) which contains 2 molequivalents of water. Then potassium tert – butoxide (560 mg, 5 mmol) is added in portions and the mixture stirred for 8 hours at room temperature. Thereupon the solvent is evaporated under vacuum after neutralization with 6 molequivalents of glacial acetic acid and the residue partitioned between ethyl acetate and aqueous potassium carbonate solution. The water phase is separated, neutralized with hydrochloric acid under cooling, the precipitate filtered off, washed with water and dried in the oven under vacuum. Thus 234 mg of pure title compound are obtained correponding to 80% yield.
  NMR ($CDCl_3$ δ ): 1.85 – 2.45 (4H,m), 3.00(2H,t), 6.92(1H,s), 7.55(1H,s),7.00 – 7.45(7H,m).

Example 4

1 – (2′ – naphthyl – ethoxycarbonylmethyl) – 5 – (3″ – chloro propyl) – 1H – imidazole (IV, X = Cl, W = 2′ – naphthyl, R′ = Et)

A solution of 1 – (2′ – naphthyl – ethoxycarbonylmethyl) – 5 – (3″ – hydroxypropyl) – 1H – imidazole (338 mg, 1 mmol) and thionyl chloride (155 mg,1.3 mmol) in dry dichloromethane (5 ml) is heated to reflux for 2 hours. Thereupon the solution is evaporated under vacuum and the residue taken up with dichloromethane and sodium bicarbonate solution. The organic phase is washed with water, dried over sodium sulfate and evaporated under vacuum. Th raw product is chromatographed on silica gel using chloroform/methanol 1% as eluant to give 320 mg of pure title compound (90% yield).
Elemental analysis found % (calculated %):
C 67.28(67.31), H 5.81 (5.93), Cl 9.85 (9.93),N 7.70 (7.85)
NMR ($CDCl_3$ δ): 1.30 (3H,t), 1.80 – 2.20 (2H,m), 2.67(2H,t), 3.51 (2H,t), 4.30 (2H,q), 5.87 (1H,m), 6.90 (1H,s) 7.60 (1H,s), 7.10 – 8.00 (7H,m).

Example 5

1 – (2′ – naphthyl – ethoxycarbonylmethyl)5 – (3″ – hydroxypropyl) – 1H – imidazole (V, W = 2′ – naphthyl, R′ = Et).

A mixture of 4 – (3′ – hydroxypropyl) – 1H – imidazole (126 mg, 1 mmol), triethylamine (121 mg, 1.2 mmol) and dimethylcarbamyl chloride (118 mg, 1.1 mmol) in dry acetonitrile (2 ml) is refluxed for 24 hours under nitrogen. After cooling to 0 – 5 ° C triethylamine (121 mg, 1.2 mmol) and trimethylsilyl chloride (130 mg, 1.2 mmol) is added and the reaction mixture stirred for 2 hours at room temperature. After dilution with ethyl ether the insolute is filtered off, the residue washed with ethyl ether and the combined ether extracts evaporated under vacuum. The resulting raw,
4 – (3′ – trimethylsilyloxypropyl ) – 1H – imidazole – 1 – dimethylcarboxamide, is reacted with bromo – (2′ – naphthyl) – acetic acid ethyl ester (293 mg, 1 mmol) in acetonitrile (2 ml) by heating at reflux for 20 hours. Then gaseous ammonia is introduced for 15 minutes at 0 – 5 ° C and the mixture concentrated under vacuum. The residue is dissolved in N – hydrochloric acid, the resulting acid solution stirred for 30 minutes at room temperature and then washed with ethyl acetate. The pH of the acid solution is brought to 8 – 9 by addition of potassium carbonate solution and the raw product is extracted with chloroform. The organic phase is washed with water, dried with sodium sulfate and then evaporated under vacuum. The residue is submitted to column chromatography on silica gel using chloroform/methanol 2% as eluant. Thus 170 mg of title compound is obtained (50% yield).
Elemental analysis found % (calculated %):
C 70.80 (70.99),H6.42 (6.55), N8.13 (8.28).
NMR ($CDCl_3$, δ): 1.32 (3H,t), 1.70 (2H,m), 2.55 (2H,t), 3.45 (2H,t), 4.25 (2H,q), 5.85 (1H,m), 6.95 (1H,s) 7.55 (1H,s), 7.10 – 8.00 (7H,m).

Example 6

1 – (1′ – anthrylmethyl) – 5 – (3″ – chloropropyl) – 1H – imidazole (III), W = 1′ – anthryl, X = Cl)

A solution of 1 – (1′ – anthrylmethyl) – 5 – (3″ – hydroxypropyl) – IH – imidazole (316 mg, 1 mmol) and thionyl chloride (155 mg, 1.3 mmol) in dry dichloromethane (4 ml) is heated to reflux for 2 hours. Then the

solution is evaporated under vacuum and the residue taken up with dichloromethane and sodium bicarbonate solution. The organic phase is washed with water, dried over sodium sulfate and evaporated under vacuum.

The residue is chromatographed on silica gel using chloroform/methanol 1% as eluant to give 301 mg of pure title compound (90% yield).
Elemental analysis found % (calculated %):
C75.25 (75.33), H 5.75 (5.72),N8.15 (8.37),Cl 10.40(10.59)
NMR($CDCl_3$ δ): 2.00(2H,m),2.60(2H,t),3.49 (2H,t), 5.15(2H,s), 6.91(1H,s), 7.60(1H,s), 7.20 – 8.20 (9H,m)

Example 7

1 – (1′ – anthrylmethyl) – 5 – (3″ – hydroxypropyl) – 1H – imidazole (VIII, W = 1′ – anthryl)

A mixture of 4 – (3′ – trimethylsilyloxypropyl) – 1H – imidazole – 1 – dimethylcarboxamide (269 mg, 1 mmol), which is prepared according to example 5, 1 – anthrylmethylbromide (271 mg, 1 mmol) and acetronitrile (2 ml) is heated at reflux for 20 hours. After cooling to 0 – 5 ° C gaseous ammonia is introduced for 15 minutes and then the mixture concentrated under vacuum.

The solution of the residue in N – hydrochloric acid is stirred for 30 minutes at room temperature and then washed with ethyl acetate. The pH is regulated to 8 – 9 by addition of potassium carbonate solution and the raw product is extracted with chloroform. The organic phase is washed with water, dried with sodium sulfate and then evaporated under vacuum. The residue is submitted to column chromatography on silica gel using chloroform/methanol 1% as eluant. Thus 221 mg (70% yield) of title compound is obtained.
Elemental analysis found % (calculated %):
C79.65 (79.72),H 6.25 (6.37), N 8.77 (8.55)
NMR ($CDCl_3$, δ):1.81 (2H,m),2.55 (2H,t),3.45(2H,t), 5.25(2H,s),6.85 (1H,s),7.60 (1H,s), 7.15 – 8.15 (9H,m).

Example 8

5 – (2′ – naphthyl) – 5,6,7,8 – tetrahydroimidazo[1.5 – a] pyridine hydrpchloride (I, W = 2′ – naphthyl)

In order to obtain the hydrochloride salt to a solution of 5 – (2′ – naphtyl) – 5,6,7,8 – tetrahydroimidazo [1.5 – a] pryidine base (258 mg, 1 mmol) in ethyl acetate (4 ml) a stoichiometric amount of hydrochloric acid is added. The precipitated salt is filtered off, washed with solvent and dried. Thus the title compound is obtained in 90% yield (26.5 mg).
Elemental analysis found % (calculated %):
C71.65 (71.70), H5.95 (6.02),N 9.78(9.84),Cl 12.33(12.45)

Example 9

Tablets, each weighing 0.150 g and containing 25 mg of the active substance, can be manufactured as follows:

| | |
|---|---|
| 5 – (2′ – naphthyl) – 5,6,7,8 – tetrahydroimidazo [1.5 – a] pyridine | 250 g |
| Lactose | 800 g |
| Corn starch | 415 g |
| Talc powder | 30 g |
| Magnesium stearate | 5 g |

The 5 – (2′ – naphthyl) – 5,6,7,8 – tetrahydroimidazo [1.5 – a] pyridine, the lactose and half the corn starch are mixed; the mixture is then forced through a sieve of 0.5 mm mesh size.

Corn starch (10 g) is suspended in warm water (90 ml) and the resulting paste is used to granulate the powder. The granulate is dried, comminuted on a sieve of 1.4 mm mesh size, then the remaining quantity of starch, talc and magnesium stearate is added, carefully mixed and processed into tablets.

## Claims

1. A compound having the following general formula (I)

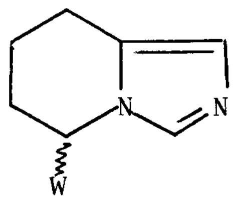

(I)

wherein W is
a 2-naphthyl or 1- or 2- or 9-anthryl radical wherein each benzene ring is unsubstituted or substituted by a substituent chosen from halogen, $C_1-C_4$ alkyl, phenyl, phenyl-$C_1-C_4$ alkyl, hydroxy, $C_1-C_4$ alkoxy, phenoxy, phenyl-$C_1-C_4$ alkoxy, formyloxy, $C_2-C_5$ alkanoyloxy, benzoyloxy, mercapto, $C_1-C_4$ alkylthio, phenylthio, phenyl-$C_1-C_4$ alkylthio, $C_1-C_4$ alkylsulfonyl, phenylsulfonyl, $C_2-C_5$ alkanoylthio, $C_2-C_5$ alkanoylamino, benzoylamino, $C_2-C_5$ alkanoyl, benzoyl, carboxy, $C_2-C_5$ alkoxycarbonyl, cyano, nitro, mono- or di-($C_1$ $c_4$ alkyl)carbamyl, amino, mono-($C_1-C_4$ alkyl)amino, di-($C_1-C_4$ alkyl)amino, morphoiino, piperazino and $C_1-C_4$ alkyl-N-substituted piperazino, or by a quaternary ammonium radical chosen from tri-($C_1-C_4$ alkyl)amino, N-$C_1-C_4$ alkyl-substituted morpholino, N'-$C_1-C_4$ alkyl-substituted piperazino, N''-di($C_1-C_4$ alkyl)-substituted piperazino and N',N''-di($C_1-C_4$ alkyl)-substituted piperazino, and the pharmaceutically acceptable salts thereof.

2. A compound of formula (I), according to claim 1, wherein W is a 2-naphthyl or 1 -anthryl radical in which each benzene ring is unsubstituted or substituted by a substituent chosen from halogen, hydroxy, carboxy, cyano and nitro; and the pharmaceutically acceptable salts thereof.

3. A compound selected from the group consisting of:
5-(2'-naphthyl)-5,6,7,8-tetrahydroimidazo [1.5-a] pyridine;
5-(4'-chloro-2'-naphthyl)-5,6,7,8-tetrahydroimidazo [1.5-a] pyridine;
5-(4'hydroxy-2'-naphthyl)-5,6,7,8-tetrahydroimidazo [1.5-a] pyridine;
5-(4'-carboxy-2'-naphthyl)-5,6,7,8-tetrahydroimidazo [1.5-a] pyridine;
5-(4'-cyano-2'-napthyl)-5,6,7,8-tetrahydroimidazo [1.5-a] pyridine;
5-(4'-nitro-2'naphthyl)-5,6,7,8-tetrahydroimidazo [1.5-a] pyridine;
5-(1'-anthryl)-5,6,7,8-tetrahydroimidazo [1.5-a] pyridine;
5-(4'-chloro-1'-anthryl)-5,6,7,8-tetrahydroimidazo [1.5-a] pyridine;
5-(4'-hydroxy-1'-anthryl)-5,6,7,8-tetrahydroimidazo [1.5-a] pyridine;
5-(4'-carboxy-1'-anthryl)-5,6,7,8-tetrahydroimidazo [1.5-a] pyridine;
5-(4'-cyano-1'-anthryl)-5,6,7,8-tetrahydroimidazo [1.5-a] pyridine;
5-(4'-nitro-1'-anthryl)-5,6,7,8-tetrahydroimidazo [1.5-a] pyridine;
and the pharmaceutically acceptable salts thereof.

4. A process for the preparation of a compound of formula (I) and the salts thereof, according to claim 1, the process comprising:
A) decarboxylating a compound of formula (II):

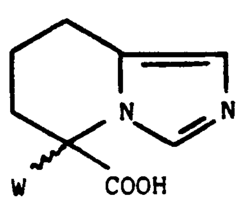

( II)

wherein W is as defined in claim 1; or

B) cyclizing a compound of formula (III):

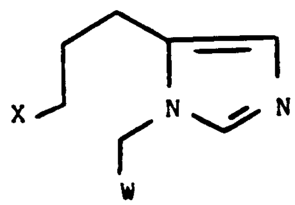

(III)

wherein W is as defined in claim 1 and X is a leaving group; and, if desired, converting a compound of formula (I) into another compound of formula (I) and/or, if desired, salifying a compound of formula (I) and/or, if desired, obtaining a free compound from a salt and/or, if desired, separating a mixture of isomers of compounds of formula (I) into the single isomers.

**5.** A pharmaceutical composition containing a suitable carrier and/or diluent and, as an active principle, a compound of formula (I) according to claim 1 or a pharmaceutically acceptable salt thereof.

**6.** The use of a compound of formula (I), or a pharmaceutically acceptable salt thereof, according to claim 1, in the preparation of a pharmaceutical composition for use as an aromatase inhibitor.

**Patentansprüche**

**1.** Eine Verbindung mit der nachstehenden allgemeinen Formel (I)

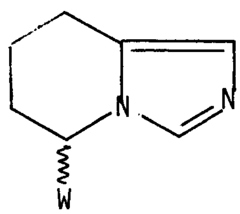

(I)

worin
W ein 2-Naphthyl- oder ein 1- oder 2- oder 9-Anthryl-Radikal bedeutet, in welchem jeder Benzolring nichtsubstituiert oder durch einen unter Halogen, $C_1-C_4$ Alkyl, Phenyl, Phenyl-$C_1-C_4$ Alkyl, Hydroxy, $C_1-C_4$ Alkoxy, Phenoxy, Phenyl-$C_1-C_4$ Alkoxy, Formyloxy, $C_2-C_5$ Alkanoyloxy, Benzoyloxy, Mercapto, $C_1-C_4$ Alkylthio, Phenylthio, Phenyl-$C_1-C_4$ Alkylthio, $C_1-C_4$-Alkylsulfonyl, Phenylsulfonyl, $C_2-C_5$ Alkanoylthio, $C_2-C_5$ Alkanoylamino, Benzoylamino, $C_2-C_5$ Alkanoyl, Benzoyl, Carboxy, $C_2-C_5$ Alkoxycarbonyl, Cyano, Nitro, mono- oder di-($C_1-C_4$ Alkyl)carbamyl, Amino, mono-($C_1-C_4$ Alkyl)amino, di-($C_1-C_4$ Alkyl)amino, Morpholino, Piperazino und $C_1-C_4$ Alkyl-N-substituiertem Piperazino ausgewählten Substituenten, oder durch ein unter tri($C_1-C_4$ Alkyl)amino, N-$C_1-C_4$ Alkyl-substituiertem Morpholino, N'-$C_1-C_4$ Alkyl-substituiertem Piperazino, N''-di($C_1-C_4$ Alkyl)-substituiertem Piperazino und N',N''-di($C_1-C_4$ Alkyl)-substituiertem Piperazino ausgewähltes quaternäres Ammoniumradikal substituiert ist, und die pharmazeutisch annehmbaren Salze derselben.

**2.** Eine Verbindung der Formel (I), gemäss Anspruch 1, worin W ein 2-Naphthyl- oder ein 1-Anthryl-Radikal bedeutet, in welchem jeder Benzolring nichtsubstituiert oder durch einen unter Halogen, Hydroxy, Carboxy, Cyano und Nitro ausgewählten Substituenten substituiert ist, und die pharmazeutisch annehmbaren Salze derselben.

**3.** Eine von der nachstehend angegebenen Gruppe ausgewählte Verbindung:
5-(2'-Naphthyl)-5,6,7,8-tetrahydroimidazo [1.5-a] Pyridin;
5-(4'-Chlor-2'-naphthyl)-5,6,7,8-tetrahydroimidazo [1.5-a] Pyridin;
5-(4'-Hydroxy-2'-naphthyl)-5,6,7,8-tetrahydroimidazo [1.5-a] Pyridin;
5-(4'-Carboxy-2'-naphthyl)-5,6,7,8-tetrahydroimidazo [1.5-a] Pyridin;
5-(4'-Cyano-2'-naphthyl)-5,6,7,8-tetrahydroimidazo [1.5-a] Pyridin;

5-(4'-Nitro-2'-naphthyl)-5,6,7,8-tetrahydroimidazo [1.5-a] Pyridin;
5-(1'-Anthryl)-5,6,7,8-tetrahydroimidazo [1.5-a] Pyridin;
5-(4'-Chlor-1'-anthryl)-5,6,7,8-tetrahydroimidazo [1.5-a] Pyridin;
5-(4'-Hydroxy-1'-anthryl)-5,6,7,8-tetrahydroimidazo [1.5-a] Pyridin;
5-(4'-Carboxy-1'-anthryl)-5,6,7,8-tetrahydroimidazo [1.5-a] Pyridin;
5-(4'-Cyano-1'-anthryl)-5,6,7,8-tetrahydroimidazo [1.5-a] Pyridin;
5-(4'-Nitro-1'-anthryl)-5,6,7,8-tetrahydroimidazo [1.5-a] Pyridin;
und die pharmazeutisch annehmbaren Salze derselben.

**4.** Ein Verfahren zur Herstellung einer Verbindung der Formel (I) und der Salze derselben, gemäss Anspruch 1, dadurch gekennzeichnet, dass es

A) die Dekarboxylierung einer Verbindung der Formel (II):

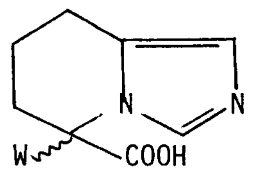

(II)

worin W die im Anspruch 1 angegebene Bedeutung hat; oder

B) die Zyklisation einer Verbindung der Formel (III):

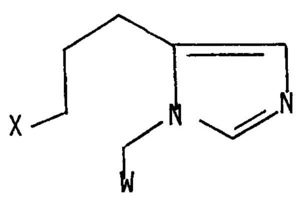

(III)

worin W die im Anspruch 1 angegebene Bedeutung hat und X eine ausscheidende Gruppe ist, und, sofern dies gewünscht wird, die Umwandlung einer Verbindung der Formel (I) in eine andere Verbindung der Formel (I) und/oder, sofern dies gewünscht wird, die Verwandlung einer Verbindung der Formel (I) in ein Salz und/oder, sofern dies gewünscht wird, das Erhalten einer freien Verbindung von einem Salz und/oder, falls dies gewünscht wird, das Auflösen eines Isomergemisches der Verbindungen der Formel (I) in die einzelnen Isomere, umfasst.

**5.** Eine pharmazeutische Zusammensetzung, dadurch gekennzeichnet, dass sie einen passenden Träger und/oder Verdünner und, als Wirkstoff, eine Verbindung der Formel (I) gemäss Anspruch 1 oder ein pharmazeutisch annehmbares Salz derselben enthält.

**6.** Die Anwendung einer Verbindung der Formel (I) oder ein pharmazeutisch annehmbares Salz derselben, gemäss Anspruch 1, für die Herstellung einer pharmazeutischen Zusammensetzung zum Gebrauch als Aromatase-Inhibitor.

## Revendications

**1.** Un composé représenté par la formule générale (I) suivante:

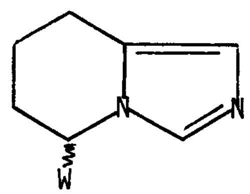

(I)

dans laquelle
W représente un radical 2-naphthyle ou 1- ou 2- ou 9-anthryle dans lequel chacun des anneaux de benzène n'est pas substitué ou est substitué par un substituant choisi parmi halogène, $C_1-C_4$ alkyle, phényle, phényl-$C_1-C_4$ alkyle, hydroxy, $C_1-C_4$ alcoxy, phénoxy, phényl-$C_1-C_4$ alcoxy, formyloxy, $C_2-C_5$ alcanoyloxy, benzoyloxy, mercapto, $C_1-C_4$ alkylthio, phénylthio, phényl $C_1-C_4$ alkylthio, $C_1-C_4$ alkylsulfonyle, phénylsulfonyle, $C_2-C_5$ alcanoylthio, $C_2-C_5$ alcanoylamino, benzoylamino, $C_2-C_5$ alcanoyle, benzoyle, carboxy, $C_2-C_5$ alcoxycarbonyle, cyano, nitro, mono- ou di-($C_1-C_4$ alkyl)carbamyle, amino, mono-($C_1-C_4$ alkyl)amino, di-($C_1-C_4$ alkyl)amino, morpholino, pipérazino et pipérazino substitué par $C_1-C_4$ alkyl-N, ou par un radical d'ammonium quaternaire choisi parmi tri-($C_1-C_4$ alkyl)amino, morpholino substitué par N-$C_1-C_4$ alkyle, pipérazino substitué par N'-$C_1-C_4$ alkyle, pipérazino substitué par N''-di($C_1-C_4$ alkyle) et pipérazino substitué par N',N''-di($C_1-C_4$ alkyle), et les sels de ces derniers acceptables du point de vue pharmaceutique.

**2.** Un composé de la formule (I), selon la revendication 1, dans laquel: W représente un radical 2-naphthyle ou 1-anthryle dans lequel chacun des anneaux de benzène n'est pas substitué ou est substitué par un substituant choisi parmi halogène, hydroxy, carboxy, cyano et nitro; et les sels de ces derniers acceptables du point de vue pharmaceutique.

**3.** Un composé à choix du groupe consistant en:
5-(2'-naphthyl)-5,6,7,8-tétrahydroimidazo [1.5-a] pyridine;
5-(4'-chloro-2'-naphthyl)-5,6,7,8-tétrahydroimidazo [1.5-a] pyridine;
5-(4'-hydroxy-2'-naphthyl)-5,6,7,8-tétrahydroimidazo [1.5-a] pyridine;
5-(4'-carboxy-2'-naphthyl)-5,6,7,8-tétrahydroimidazo [1.5-a] pyridine;
5-(4'-cyano-2'-naphthyl)-5,6,7,8-tétrahydroimidazo [1.5-a] pyridine;
5-(4'-nitro-2'-naphthyl)-5,6,7,8-tétrahydroimidazo [1.5-a] pyridine;
5-(1'-anthryl)-5,6,7,8-tétrahydroimidazo [1.5-a] pyridine;
5-(4'-chloro-1'-anthryl)-5,6,7,8-tétrahydroimidazo [1.5-a] pyridine;
5-(4'-hydroxy-1'-anthryl)-5,6,7,8-tétrahydroimidazo [1.5-a] pyridine;
5-(4'-carboxy-1'-anthryl)-5,6,7,8-tétrahydroimidazo [1.5-a] pyridine;
5-(4'-cyano-1'-anthryl)-5,6,7,8-tétrahydroimidazo [1.5-a] pyridine;
5-(4'-nitro-1'-anthryl)-5,6,7,8-tétrahydroimidazo [1.5-a] pyridine;
et les sels de ces derniers acceptables du point de vue pharmaceutique.

**4.** Un procédé de fabrication d'un composé de la formule (I) et des sels de ce dernier selon la revendication 1, caractérisé par le fait qu'il comprend:
A) la décarboxylation d'un composé de la formule (II):

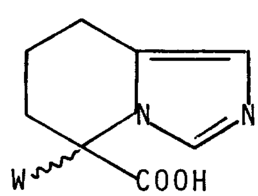

(II)

dans laquelle W est tel que défini à la revendication 1; ou
B) la cyclisation d'un composé de la formule (III):

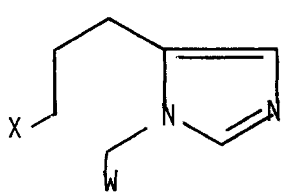

(III)

dans laquelle W est tel que défini à la revendication 1 et X représente un groupe sortant; et, pour autant qu'on le souhaite, la conversion d'un composé de la formule (I) en un autre composé de la

formule (I) et/ou, pour autant qu'on le souhaite, la salification d'un composé de la formule (I) et/ou, pour autant qu'on le souhaite, l'obtention d'un composé libre d'un sel et/ou, pour autant qu'on le souhaite, la résolution d'un mélange d'isomères dans les isomères singuliers.

**5.** Une composition pharmaceutique caractérisée par le fait qu'elle contient un support et/ou un diluant et, comme substance active, un composé de la formule (I) selon la revendication 1 ou l'un de ses sels acceptables du point de vue pharmaceutique.

**6.** L'emploi d'un composé de la formule (I), ou de l'un de ses sels, selon la revendication 1, pour la préparation d'une composition pharmaceutique à employer comme inhibiteur de l'aromatase.